# EUROPEAN PATENT APPLICATION

(11) **EP 4 492 200 A1**
(43) Date of publication of application: **15.01.2025**
(21) Application number: 22930714.5
(22) Date of filing: 07.03.2022
(51) Int. Cl.: G06F 3/01

(54) **SKIN STIMULATION DEVICE AND METHOD FOR DRIVING SKIN STIMULATION DEVICE**

(71) Applicant: Sone, Junji, Chigasaki-shi, Kanagawa 253-0012 (JP)
(72) Inventor: LIN, Liwei, Berkeley, CA 94720-1740 (US); SONE, Junji, Chigasaki-shi, Kanagawa 253-0012 (JP)
(74) Representative: Rings, Rolf
(86) International application number: PCT/JP2022/009620
(87) International publication number: WO 2023/170728

(57) **Abstract**

A skin stimulation device that applies stimulation to the skin including a first vibrating member that vibrates by being vibrated or vibrates by itself, a vibration film that contacts the skin and transmits the vibration as stimulation, and a first support member supporting the vibrating film on a first surface, which is a predetermined surface, and supporting the first vibrating member on a second surface opposite the first surface, wherein a first hole penetrating from the first surface to the second surface is formed, and, in the first hole, a first opening on the first surface has a narrower area than a second opening on the second surface, wherein the vibration film is supported by the first support member so as to close the first opening, and the first vibrating member is supported by the first support member so as to close the second opening and is vibrated or vibrates itself in the position of closing the second opening.

## Description

### Technical Field

This invention relates to skin stimulation devices and methods of driving skin stimulation devices, and in particular, to skin stimulation devices that apply stimulation to the skin and methods of driving skin stimulation devices.

### Background Technology

Tactile and force feedback are important for surgical systems and teleoperation tasks. There are numerous products for Braille displays, and tactile devices have been studied. Tactile displays require high density and fast response to achieve the teleoperation and virtual reality that braille codes display. One of these tactile displays has been realized using magnetic microactuators based on PDMS (polydimethylsiloxane) elastomers.

Although these tactile displays have achieved high density and high speed, the forces generated are weak. Ion-conductive polymer gel membrane actuators have achieved high density and high speed displays in wet conditions, but wet conditions are problematic. When dry, they stop working because the ions do not move.

Shape memory alloy actuators are one possible solution, but they do not have sufficient response time for use in tactile devices. High-resolution displays using smart hydrogels have also been developed, but the response time is insufficient for tactile sensation. Similar polymer actuators have been developed using dielectric elastomers to achieve soft, flexible, thin actuators that can be used in wearable devices, but cannot present pressure sensation.

Tactile generation devices that array multiple ultrasonic transducers create tactile sensation at or near a single point of vibration stimulation from multiple transducers at a distant distance, but they are large devices that require high-power drives, making it difficult to generate tactile sensation at multiple points. Some pseudo-haptic devices use electrical stimulation and change tactile sensation according to polarity (see, for example, Non-Patent Document 1). Others use air jets or suction, but are difficult to use as wearable devices because the devices are large and interfere with hand work.

### Prior art document

### Patent documents

Non-Patent Document 1: H. Kajimoto, Electro-tactile display with real-time impedance feedback using pulse width modulation, IEEE Trans. Haptics, 5 (2), (2012), 184- 188.

### Summary of Invention

### Problem to be solved by the invention

Thus, tactile sensation is created mainly by vibration and pin up/down, but due to low generation density, slow response speed, and large mechanical structure of the drive mechanism, it was difficult to attach the device to the human body without interfering with a person's free work.

The present invention was made in view of this situation, and is a wearable device that allows stimulation to be applied to a desired part of the skin area at a desired time and for a desired period of time, causing a tactile sensation.

### Means to solve the problem

A skin stimulation device includes: a first vibration member that is vibrated by excitation or that vibrates by itself; a vibration film that makes contact with the skin and transmits vibration as stimuli; and a first support member that supports the vibration film by a predetermined first surface and supports the first vibration member by a second surface opposing the first surface, and that has formed therein a first hole which penetrates from the first surface to the second surface and in which the area of a first opening on the first surface side is smaller than the area of the second opening on the second surface side. The vibration film is supported by the first support member to close the first opening. The first vibration member is supported by the first support member to close a second opening.

A plurality of first holes can be formed in the first support member.

The spacing between the plurality of first holes in the first support member can be less than the two-point identification threshold of human skin.

A vibratory means for vibrating the first vibrating member can be further provided, which is provided in contact with the first vibrating member at the position where the first vibrating member is closing the second opening.

The vibratory means can be made to resonate at a predetermined frequency.

The vibratory means can be a piezoelectric element.

A second vibrating member that vibrates by itself can be further provided, which is provided with a spacer between it and the first vibrating member that vibrates by itself.

The first and second vibration members can be piezoelectric polymer films.

A coating can be formed on the inner surface of the first hole of the first support member with a material of greater density compared to the density of the first support member.

A second support member can be further provided between the first support member and the first vibration member, molded of a material of lesser hardness compared to the hardness of the first support member, and having a second hole of the same cross-sectional shape as the second opening that opens at the location of the second opening.

When the vibrating film contacts the skin and transmits vibration as stimulation, further pressing means can be provided to displace and press the first vibrating member, vibrating film and first support member against the skin.

The pressing means can be made to press against the skin by displacing the first vibrating member, the vibrating film, and the first support member by gas or liquid pressure.

The pressing means can be used with a cam to displace the first vibrating member, the vibrating film and the first support member to press them against the skin.

The pressing means can be pulled by a wire through a pulley to displace the first vibrating member, vibrating film and first support member to press them against the skin.

A method of driving a skin stimulation device in one aspect of the invention is a method of driving a skin stimulation device that applies stimulation to skin, comprising: a vibrating member that vibrates by being shaken or vibrates by itself, a vibrating film that contacts skin and transmits vibration as stimulation, supporting the vibrating film on a first surface, which is a predetermined surface, and Supporting the vibrating member at a second surface opposite the first surface, the support member including a hole penetrating from the first surface to the second surface, the hole having an area of the first opening on the first surface side narrower than the area of the second opening on the second surface side, the vibrating film being supported by the support member to close the first opening, the vibrating member being The vibrating member of the skin stimulation device is supported by the support member so as to close the second opening and is vibrated in the position in which the second opening is closed or is vibrated by itself, in the form of a burst signal that is turned on or off at a period of 3 msec to 30 msec and, when on, vibrates the vibrating member at a frequency that causes it to resonate.

### Effects of the Invention

As described above, the present invention enables a wearable device to apply stimulation causing tactile and pressure sensations to a desired part of the skin site at a desired time and for a desired period of time.

### Brief Description of Drawings

Fig. 1 shows a block diagram of each level of the tactile sensation presentation system 11, including a tactile sensation generating pad, which is an example of a skin stimulation device.
Fig. 2 shows an example of the configuration of the tactile sensation generating pad group 21.
Fig. 3 shows another example of the configuration of the tactile sensation generating pad group 21.
Fig. 4 shows another example of the configuration of the tactile sensation generating pads 21 is shown.
Fig. 5 shows an enlarged cross-sectional view of a part of the cross section of the tactile sensation generating pad 73.
Fig. 6 shows an enlarged cross-sectional view of a part of the cross-section of the tactile sensation generating pad 251.
Fig. 7 shows an enlarged cross-sectional view of tactile sensation generating pad 301.
Fig. 8 shows a state of the tactile sensation generating pad 73 attached to the finger abdomen.
Fig. 9 shows an example of resonance characteristics of the vibration actuator 202 or piezoelectric polymer films 311-1 and 311-2.
Fig. 10 shows an example of the waveform of the voltage supplied from the amplifier 35 to the tactile generation pad 73, the tactile generation pad 251 or the tactile generation pad 301.
Fig. 11 shows an example of the waveform of the voltage supplied from the amplifier 35 to the tactile sensation generation pad 73, the tactile sensation generation pad 251 or the tactile sensation generation pad 301.

### Forms for carrying out the invention

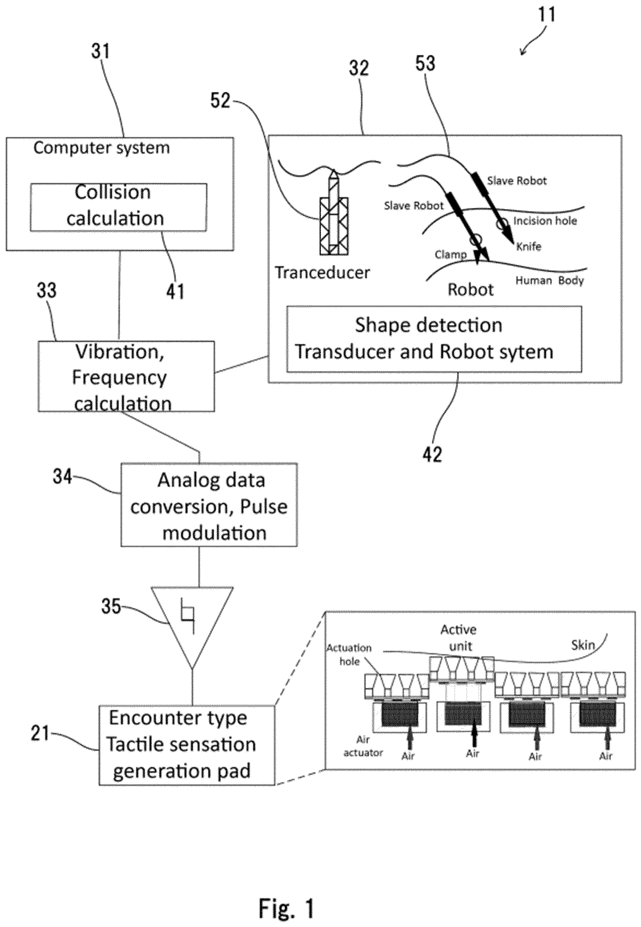

The following is a description of the skin stimulation device of the present invention with reference to Figures 1 through 11.

Figure 1 shows a block diagram of each level of the tactile presentation system 11, including a tactile generating pad, which is an example of a skin stimulation device. The tactile presentation system 11 includes a virtual reality computer system and a teleoperation system. The tactile presentation system 11 produces calculated or detected tactile sensations in humans. That is, the tactile presentation system 11 calculates or detects tactile sensations and applies stimuli to human skin to perceive the calculated or detected tactile sensations. The skin to which the stimulation is applied can be human or animal.

The tactile presentation system 11 comprises a computer system 31, a tactile detection system 32, a vibration frequency and pressure calculation unit 33, a digital data/analog data conversion unit 34, an amplifier 35, and a group of tactile generating pads 21. Computer system 31 includes and comprises a computer to calculate contact or collision with the skin. Computer system 31 includes a collision calculation section 41. The collision calculation section 41 is implemented by a computer executing a predetermined program to calculate human contact or collision, real or virtual. For example, the collision calculation section 41 calculates the avatar's contact or collision with other avatars or objects in the virtual space according to the avatar's movements in the virtual space. For example, the collision calculation section 41 calculates tactile information, such as tactile, pressure or vibration sensations of other avatars or objects when the avatar contacts or collides with other avatars or objects in the virtual space. Computer system 31 supplies the calculated tactile information to vibration frequency and pressure calculation section 33.

For example, tactile and pressure sensations are sensations caused by weak mechanical stimuli applied to the skin surface. For example, tactile sensation is a sensation based on receptors in the skin that emit a large number of impulses immediately after a stimulus is applied. Pressure, for example, is a sensation felt at deep sensory arthrodeses and slow cutaneous sensory receptors (Ruffini endings). Vibratory sensation, for example, is a sensation produced by repetitive stimulation of tens to hundreds of Hz.

The tactile detection system 32 detects tactile sensations. The tactile detection system 32 includes a shape detection transducer and a robotic system 42. The shape detection transducer and robotic system 42 detects tactile sensations according to shape or tactile information at work. Shape detection transducer and robotic system 42 includes transducer 52 or remote robotic system 53. The transducer 52 traces the surface of the object and detects tactile sensations according to the shape of the object. The remote robot system 53 detects tactile information from the end-effector (tool) of a robot that is remotely operated by a human during work. For example, the remote robot system 53 detects tactile information from a tool, such as a medical teleoperated robot, which is a knife or clamp. For example, the remote robot system 53 detects tactile information such as tactile, pressure or vibration sensations of reaction forces such as skin during work, when touching a human body, sutures or tools with tools that are knives or clamps, and during work. The tactile sensation detection system 32 supplies the detected tactile information to the vibration frequency and pressure calculation unit 33.

Thus, tactile information indicating tactile, pressure or vibration sensations is generated from work simulations in a virtual reality environment, tactile data measured by a remote robot, image data, etc.

The vibration frequency/pressure calculation section 33 consists of a computer executing a predetermined program or a dedicated device, etc., and calculates the vibration amount and frequency to perceive the tactile sensation from the tactile information supplied by the computer system 31 or the tactile detection system 32. For example, if the tactile information supplied by computer system 31 or tactile detection system 32 is tactile information indicating tactile, pressure, or vibration sensation, etc., the vibration frequency/pressure calculation section 33 calculates the vibration amount and frequency corresponding to the tactile sensation, and the vibration amount and frequency corresponding to the pressure sensation and the The force presented by actuators such as cylinders or cams is calculated, and the vibration amount and frequency are calculated according to the vibration sensation. The vibration frequency and pressure calculation section 33 supplies digital data indicating the calculated vibration amount and frequency to the digital data/analog data conversion section 34.

The digital data/analog data conversion section 34 converts digital data indicating vibration amount and frequency supplied by the vibration frequency and pressure calculation section 33 into analog data. For example, the digital data/analog data conversion section 34 converts digital data indicating vibration amount and frequency into analog data by frequency modulation. The digital data/analog data conversion section 34 supplies the analog data indicating the vibration amount and frequency obtained by the conversion to the amplifier 35.

Amplifier 35 consists of a power amplifier and a control output generator, and amplifies the analog data indicating the vibration amount, frequency, and pressure value supplied from digital data/analog data converter 34 to generate a control output that presses the corresponding tactile sensation generating pad against the skin from among a plurality of tactile sensation generating pads and supplies it to tactile sensation The control output is supplied to the tactile sensation generating pad group 21. Amplifier 35 is equipped with a burst transmission function, as described in detail below. Amplifier 35 turns on and off at predetermined cycles, and during the off period, it is approximately 0 V. During the on period, it supplies a sine wave with a predetermined voltage amplitude and a predetermined frequency to the tactile sensation generating pad group 21.

The tactile sensation generating pad group 21 is composed of a plurality of tactile sensation generating pads and an actuator that presses a given tactile sensation generating pad against the skin. The tactile sensation generating pad group 21 is created by MEMS (Micro Electro Mechanical Systems), ultra-precision machining or 3D printing technology (3D modeling technology). The tactile pads 21 are attached to the skin of the body, such as fingers, palms, backs of hands, wrists, elbows, shoulders, chest, back, or hips, by means of a fixation band, harness, gloves, or clothing. For example, the shape of the tactile sensation generating pad group 21 is a surface shape corresponding to the palm side surface of the terminal segment of the second finger (index finger) and is between 3 mm and 5 mm thick. For example, the shape of the tactile sensation generating pad group 21 is a surface corresponding to the palm side surface of the right hand, and is between 3 mm and 5 mm thick. The tactile sensation generating pad group 21 is driven by the amplifier 35 to generate stimuli that produce tactile, pressure, or vibration sensations and transmit the stimuli to the skin at the desired site among the skin sites. The tactile generating pad group 21 applies stimuli that cause tactile, pressure or vibration sensations to the desired area of the skin at the desired timing. In other words, the tactile generating pad group 21 applies stimuli that cause tactile and pressure sensations to the desired area of skin, among the areas of skin, at the desired time and for the desired duration.

The tactile generating pad group 21 can selectively generate stimuli that produce tactile sensations over one or more of the tactile generating pads 73, which are tactile presentation units described below, in response to tactile information, and can also produce pressure sensations simultaneously.

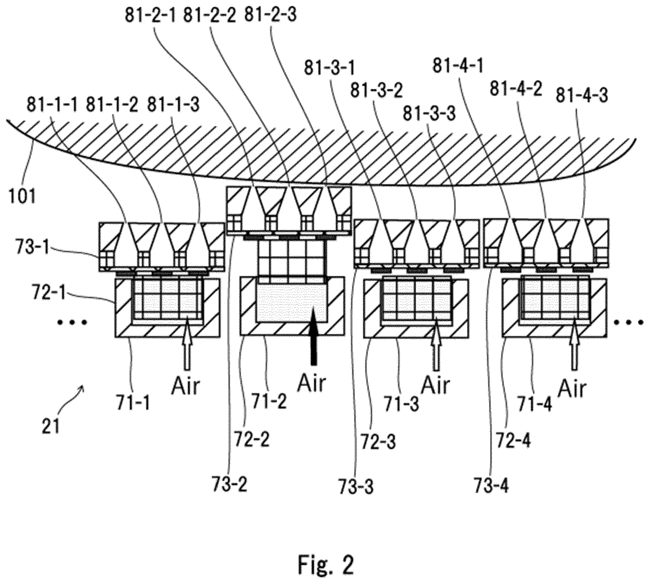

Figure 2 shows an example of the configuration of the tactile sensation generating pad group 21. The tactile sensation generating pad group 21 is composed of about 3 to 10 tactile sensation generating pads 73. Each of the tactile generating pads 73 has from 10 to 50 tactile generating points. The tactile generating points on the tactile generating pad 73 are arranged in the left-right direction in Fig. 2 and the front-back direction (depth direction) in Fig. 2. In other words, the tactile sensation generating points are arranged two-dimensionally on the surface at predetermined intervals on the skin-contacting side of the tactile sensation generating pad 73.

In the following description, some of the tactile generating points of the tactile generating pads 73 will be used as examples. When the tactile sensation generating pad group 21 transmits stimuli to the skin with a predetermined tactile sensation generating pad 73 among a plurality of tactile sensation generating pads 73 that are lined up, the predetermined tactile sensation generating pad 73 is moved in the direction of the skin 101 so that it contacts a predetermined part of the skin 101 of the body such as fingers, hands, or arms.

Hereafter, for example, when four tactile generation pads 73 are included in the tactile generation pad group 21, when distinguishing the tactile generation pads 73 individually, each is referred to as a tactile generation pad 73-1 through 73-4, etc.

Figure 2 shows an example of the configuration of the tactile sensation generating pad group 21 when four tactile sensation generating pads 73-1 through 73-4 are included. The tactile sensation generating units 71-1 through 71-4 are configured to include each of the air actuators 72-1 through 72-4 and the tactile sensation generating pads 73-1 through 73 4 are configured to include each of the air actuators 72-1 through 72-4 and the tactile sensation generating pads 73-1 through 73-4, respectively. The tactile sensation generating units 71-1 through 71-4 each operate individually and independently to generate stimuli and transmit the generated stimuli to the skin 101.

The tactile sensation generating pad 73-1 has holes formed through the surface of the tactile sensation generating pad 73-1 that are in contact with the skin 101 and the surface opposite the surface in contact with the skin 101, as will be described in detail below. A plurality of holes are formed in the tactile sensation generating pad 73-1. Apertures 81-1-1 through 81-1-3 of the surface of the tactile sensation generating pad 73-1 that is in contact with the skin 101 lead to each of the through holes. Similarly, each of the tactile sensation generating pads 73-2 through 73-4 has two sides of the surface of each of the tactile sensation generating pads 73-2 through 73-4 that are in contact with the skin 101 and two sides of the surface of each of the tactile sensation generating pads 73-2 through 73-4 that are in contact with the skin 101, as will be described in detail below. The holes are formed through the side in contact with the skin 101 and the side opposite the side in contact with the skin 101. A plurality of holes are formed in each of the tactile sensation generating units 71-2 through 71-4. Apertures 81-2-1 through 81-2-3 on the side of the face of the tactile sensation generating pad 73-2 that is in contact with the skin 101 lead to each of the through holes. Openings 81-3-1 through 81-3-3 on the side of the face of tactile sensation generating pad 73-3 that is in contact with skin 101 lead to each of the through holes. Openings 81-4-1 through 81-4-3 in the side of the face of the tactile sensation generating pad 73-4 that is in contact with the skin 101 lead to each of the through holes.

In tactile generating pads 73-1 through 73-4, apertures 81-1-1 through 81-1-3, apertures 81-2-1 through 81-2-3, apertures 81-3-1 through 81-3-3 and apertures 81-3-1 through 81-3-3 and apertures 81-3-1 through 81-3-3. 81-1-1 to 81-2-3, 81-2 to 81-2-3, 81-3-1 to 81-3-3, and 81-4-1 to 81-4-3, respectively, in tactile generating pads 73-1 to 73-4. 81-4-1 through 81-4-3 are tactile generating points that produce stimuli that cause tactile and pressure sensations, respectively. Apertures 81-1-1 through 81-1-3 and 81-2-1 through 81-2-3, apertures 81-3-1 through 81-3-3, and apertures 81-4-1 through 81-4-3. The spacing between apertures 81-2-1 through 81-2-3, 81-3-1 through 81-3-3, and 81-4-1 through 81-4-3 is less than the two-point discrimination threshold of human skin. That is, apertures 81-1-1 through 81-1-3, apertures 81-2-1 through 81-2-3, apertures 81-3-1 through 81-3-3, and apertures 81-4-1 through 81-4-3 are less than the two-point discrimination threshold of human skin. 3, apertures 81-3-1 through 81-3-3, and apertures 81-4-1 through 81-4-3. The mutual distance between apertures 81-2-1 through 81-2-3, 81-3-1 through 81-3-3, and 81-4-1 through 81-4-3 is set to be less than the two-point discrimination threshold of human skin. For example, the two-point discrimination threshold of human skin is 3 to 6 mm at the fingertips and 15 to 20 mm at the palms or soles. For example, the two-point discrimination threshold for human skin is 2 to 3 mm at the lips and 30 mm at the palms or soles.

That is, for example, when tactile generating pads 73-1 through 73-4 are used for fingertips, apertures 81-1-1 through 81-1-3, apertures 81-2-1 through 81-2-3, apertures 81-3-1 through 81-3-3, and apertures 81-3-1 through 81-3-3. 81-2-1 through 81-2-3, 81-3-1 through 81-3-3, and 81-4-1 through 81-4-1 through 81-4-3. 81-4-1 through 81-4-3 are less than 3 mm apart from each other. For example, when tactile generating pads 73-1 through 73-4 are used on the palms or soles of the feet, apertures 81-1-1 through 81-1-3, apertures 81 81-2-1 through 81-2-3, 81-3-1 through 81-3-3 and 81-4-1 through 81-4-1 through 81-4-1. 81-4-1 through 81-4-3 are less than 30 mm apart from each other.

The two-point discrimination threshold of human skin is 2 to 3 mm at the lips, which is the shortest on the body, so apertures 81-1-1 to 81-1-3, 81-2-1 to 81-2-3, 81-3-1 to 81-3-3, and 81-3-1 to 81-3-3. 81-1-1 through 81-2-3, 81-3-1 through 81-3-3, and 81-4-1 through 81-4-1 through 81-2-3. 81-4-1 through 81-4-3 are less than 2 mm from each other, the tactile pads 73-1 through 73-4 can be used on any part of the body. can be used on any part of the body.

In addition, the following is a list of apertures 81-1-1 through 81-1-3, 81-2-1 through 81-2-3, 81-3-1 through 81-3-3, and 81-4-1 through 81-4-1 through 81-3-3. 81-1-1 through 81-1-3, 81-2-1 through 81-2-3, 81-3-1 through 81-3-3, and 81-4-1 through 81-4-3. 81-4-1 through 81-1-3, 81-2-1 through 81-2-3, 81-3-1 through 81-3-3, and 81-4-1 through 81-4-1 through 81-4-3.

The tactile generating pads 73-1 through 73-4 are moved in the direction of the skin 101 by each of the air actuators 72-1 through 72-4, respectively, to contact a predetermined site on the skin 101. The air actuators 72-1 through 72-4 are moved in the direction of the skin 101 by the air actuators 72-1 through 72-4, respectively. The air actuators 72-1 through 72-4 are pneumatic actuators comprising a cylinder and a piston, respectively. When compressed air of a predetermined pressure is supplied to the air actuators 72-1 through 72-4, the piston protrudes from the cylinder to displace one of the tactile sensation generating pads 73-1 through 73-4 fixed to the piston, respectively. -4, which are fixed to the piston, are displaced, thereby moving them in the direction of the skin 101. For example, at a predetermined time, air actuator 72-2 is supplied with compressed air at a predetermined pressure to displace tactile sensation generating pad 73-2, thereby moving it in the direction of skin 101, and air actuators 72 1, air actuator 72-3 and air actuator 72-4 are not supplied with compressed air at a predetermined pressure, so that tactile sensation generating pad 73-1, tactile sensation generating pad 73-3 and tactile sensation 73-4 are not moved in the direction of the skin 101. In this case, the tactile generating pad 73-2 generates stimuli and transmits the stimuli to the skin 101.

At a predetermined time, all of the tactile generating pads 73-1 through 73-4 or any one or more of the tactile generating pads 73-1 through 73-4 are moved by each of the air actuators 72 Each of the air actuators 72-1 through 72-4 is moved in the direction of the skin 101 to contact a predetermined area of the skin 101, or any of the tactile generating pads 73-1 through 73-4 are moved away from the skin 101, moved away from the skin 101.

When there is no need to distinguish between tactile sensation generating units 71-1 through 71-4 individually, they are hereinafter simply referred to as tactile sensation generating units 71. When there is no need to distinguish between air actuators 72-1 through 72-4 individually, they will hereinafter be referred to simply as air actuators 72.

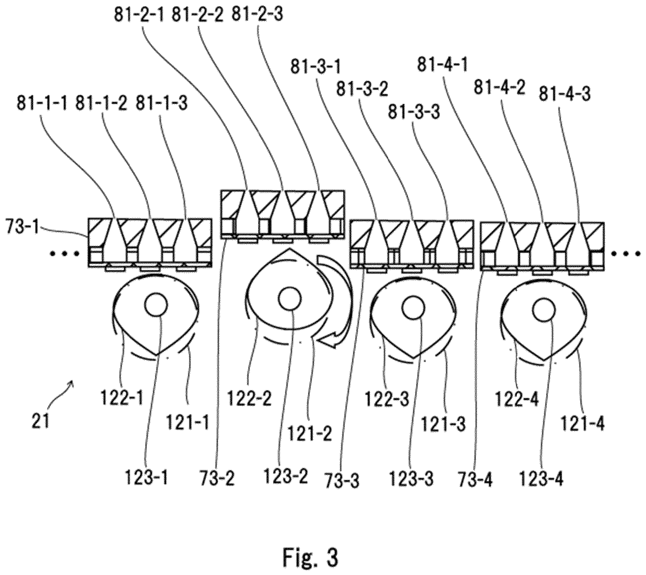

Figure 3 shows another example of the configuration of the tactile sensation generating pad group 21 when four tactile sensation generating pads 73-1 through 73-4 are included. The tactile sensation generating units 121-1 through 121-4 each include a tactile sensation generating pad 73-1 through 73-4 each, a cam 122-1 through 122 each of cams 122-1 through 122-4, and motors 123-1 through 123-4, respectively. The tactile generating units 121-1 through 121-4 each operate individually and independently to generate stimuli and transmit the generated stimuli to the skin 101.

The tactile generating pads 73-1 through 73-4 in Fig. 3 are moved in the direction of the skin 101 to contact a predetermined area of the skin 101 by each of the cams 122-1 through 122-4 and the motors 123-1 through 123 4 are moved in the direction of the skin 101 by each of the cams 122-1 through 122-4 and the motors 123-1 through 123-4, respectively, so as to contact a predetermined area of the skin 101. For example, the cams 122-1 through 122-4 are each a rotating plate cam. For example, the cams 122-1 through 122-4 each comprise a disc that is eccentric. For example, the motors 123-1 through 123-4 are each an electric motor. That is, the cams 122-1 through 122-4 are rotated by each of the motors 123-1 through 123-4, respectively, to rotate the tactile generating pads 73-1 or 73-4, respectively, and push each of the tactile generating pads 73-1 through 73-4 onto the skin 101 when they reach a predetermined angular position with respect to each of the tactile generating pads 73-1 through 73-4, respectively.

For example, at a given time, cam 122-2 is rotated by motor 123-2 so that the side protruding from the axis of rotation of cam 122-2 moves toward the tactile sensation generating pad 73-2 The cam 122-1, cam 122-3, and cam 122-4 are rotated by motor 123-1, motor 123-3, and motor 123-4 to move in the direction of skin 101 by pushing and displacing the tactile sensation-generating pad 73-2, and the cam 122-1, cam 122-3, and cam 122-4 are rotated by motor 123-1. -1, motor 123-3 and motor 123-4 do not rotate, respectively, so that tactile generating pad 73-1, tactile generating pad 73-3 and tactile generating pad 7 3-4 are not moved in the direction of skin 101. At this time, tactile generating pad 73-2 generates stimuli and transmits the stimuli to skin 101.

At a predetermined time, all of the tactile generating pads 73-1 through 73-4 or any one or more of the tactile generating pads 73-1 through 73-4 are moved by each of the cams 122 Each of cams 122-1 to 122-4 is moved by each of cams 122-1 to 122-4 in the direction of skin 101 to contact a predetermined area of skin 101, or none of the tactile generating pads 73-1 to 73-4 is moved away from skin 101, moved away from the skin 101.

For example, the tactile sensation-generating pads 73-1 through 73-4 are attached to the skin 101 away from the skin 101 by a spring or other means not shown in the figure.

Each of the cams 122-1 through 122-4 was described as a plate cam, but may be a groove cam or a three-dimensional cam. Each of the motors 123-1 through 123-4 was described as an electric motor, but may be, but is not limited to, a pressure motor, a molecular motor, or an ultrasonic motor that uses fluid pressure.

Hereafter, when there is no need to distinguish between tactile sensation generating units 121-1 through 121-4 individually, they will simply be referred to as tactile sensation generating units 121. When there is no need to distinguish between cams 122-1 through 122-4 individually, they will hereinafter be referred to simply as cams 122. When there is no need to distinguish between motors 123-1 through 123-4 individually, they will simply be referred to as motor 123.

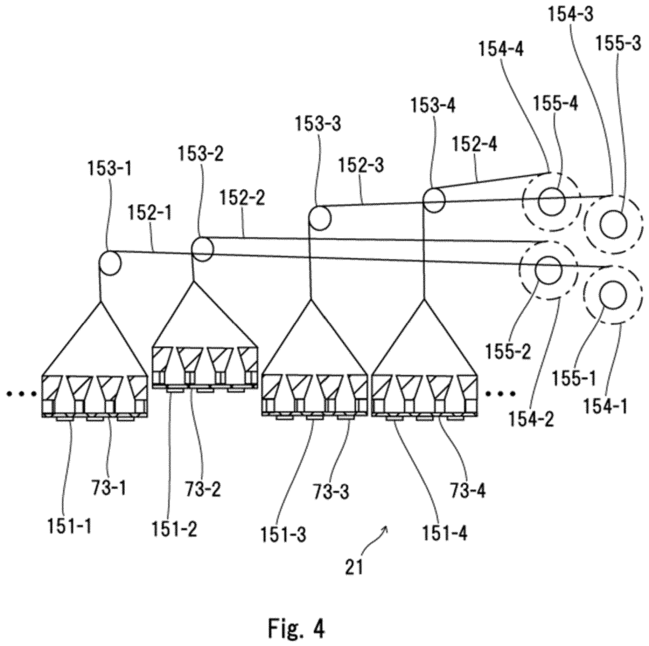

Figure 4 shows yet another example of the configuration of the tactile sensation generating pad group 21 when four tactile sensation generating pads 73-1 through 73-4 are included. The tactile sensation generating units 151-1 through 151-4 are composed of tactile sensation generating pads 73-1 through 73-4, wires 152-1 through 152 -each of pulleys 153-1 through 153-4, pulleys 154-1 through 154-4, and motors 155 155-1 through 155-4, and motors 155-1 through 155-4, respectively. Each of the tactile sensation generating units 151-1 through 151-4 operates individually and independently to generate stimuli and transmit the generated stimuli to the skin 101.

The tactile sensation generating pads 73-1 through 73-4 in FIG. 4 are each of wires 152-1 through 152-4, pulleys 153-1 through 153-4, and motors 155-1 through 155-4, respectively. 153-1 through 153-4, pulleys 154-1 through 154-4, and motors 155-1 through 155-4, respectively, to contact a predetermined area of skin 101. The wires 152-1 through 153-4 are moved in the direction of the skin 101 so as to make contact with a predetermined part of the skin 101. One end of each of the wires 152-1 through 152-4 is fixed to each of the tactile generating pads 73-1 through 73-4, and the other end of each of the wires 152-1 through 152-4 is fixed to each of the pads 73-1 through 73-4. The other end of each of the wires 152-1 through 152-4 is wound around each of the pulleys 154-1 through 154-4. Pulleys 153-1 through 153-4 are each rotatably supported on a shaft. The wires 152-1 through 152-4 are hung on each of the pulleys 153-1 through 153-4, and the direction of extension of the wires 152-1 through 152-4 is changed. The direction of extension of the wires 152-1 through 152-4 is changed. Pulleys 154-1 through 154-4 are rotated by motors 155-1 through 155-4, respectively. Motors 155-1 through 155-4 are electric motors. When each of pulleys 154-1 through 154-4 is rotated by each of motors 155-1 through 155-4, a wire wound on each of pulleys 154-1 through 154-4 is connected to the wire. When each of pulleys 154-1 through 154-4 is rotated by each of pulleys 155-1 through 155-4, the length of the wires 152-1 through 152-4 wound around each of pulleys 154-1 through 154-4 is changed, and the length of the wires 152-1 through 152-4 is changed. Each of the tactile sensation generating pads 73-1 through 73-4, which are fixed to one end of each of the wires 152-1 through 152-4, will be pulled and displaced.

That is, pulleys 154-1 through 154-4 are rotated by motors 155-1 through 155-4, respectively, to rotate each of wires 152-1 through 1 52-4, respectively, and each of the wires 152-1 through 152-4 pulls and presses each of the tactile generating pads 73-1 through 73-4 against the skin 101 against the skin 101.

For example, at a given time, pulley 154-2 is rotated by motor 155-2 to wind wire 152-2, which causes wire 152-2 to pull and displace tactile generating pad 73 -2 and displaces it in the direction of skin 101, and pulley 154-1, pulley 154-3 and pulley 154-4 are rotated by motor 155-1 motor 155-3 and motor 155-4, respectively, do not rotate, so the tactile generating pad 73-1, tactile generating pad 73-3, and tactile generating pad 73 4 are not moved in the direction of skin 101. At this time, the tactile generating pad 73-2 generates stimuli and transmits the stimuli to the skin 101.

At a given time, one or more of all of the tactile pads 73-1 through 73-4 or any one or more of the tactile pads 73-1 through 73-4 may be connected to a wire 152-1 through 152-4 via each of pulleys 153-1 through 153-4 and pulleys 154-1 through 154-4. 152-1 through 152-4 via each of pulleys 153-1 through 153-4 and pulleys 154-1 through 154-4, respectively. Each of them is pulled by each of them to move in the direction of the skin 101 so as to contact a predetermined area of the skin 101, or any of the tactile generating pads 73-1 through 73-4 are moved away from the skin 101.

For example, the tactile sensation-generating pads 73-1 through 73-4 are attached to the skin 101 away from the skin 101 by a spring or other means not shown in the figure.

For example, a group of tactile generating pads 21 including tactile generating units 151-1 through 151-4 can be realized as a glove-type device.

Motors 155-1 through 155-4 are described as electric motors, respectively, but may also be pressure motors, molecular motors, or ultrasonic motors that use fluid pressure.

Hereafter, when there is no need to distinguish between tactile sensation generating units 151-1 through 151-4 individually, they will simply be referred to as tactile sensation generating units 151. When there is no need to distinguish between wires 152-1 through 152-4 individually, they are hereinafter simply referred to as wires 152. When there is no need to distinguish between pulleys 153-1 through 153-4 individually, they are simply referred to as pulleys 153. When it is not necessary to distinguish between pulleys 154-1 through 154-4 individually, they are simply referred to as pulleys 154. When it is not necessary to distinguish between motors 155-1 through 155-4 individually, they are simply referred to as motor 155.

In addition, a linkage mechanism, such as a leverage or pantograph, can be provided between each of the tactile sensation generating pads 73-1 through 73-4 and each of the air actuators 72-1 through 72-4 or between each of the tactile sensation generating pads 73-1 through 73-4 and each of the air actuators 72-1 through 72-4. A link mechanism such as a leverage or pantograph is provided between each of the tactile sensation generating pads 73-1 through 73-4 and each of the cams 122-1 through 122-4, and a link mechanism such as a pantograph is provided between each of the air actuators 72-1 through 72-4 and each of the air actuators 72-1 through 72-4. Each of the air actuators 72-1 through 72-4 to each of the tactile pads 73-1 through 73-4, or each of the cams 122-1 through 122-4 to each of the tactile pads 73-1 through 73-4, or each of the cams 122-1 through 122-4 to each of the tactile pads 73-1 through 73-4. The driving force may be transmitted to each of the pads 73-1 through 73-4 from each of the air actuators 72-1 through 72-4 or from each of the cams 122-1 through 122-4.

In addition, air actuators 72-1 through 72-4, cams 122-1 through 122-4 and motors 123-1 through 123-4, and wires 152-1 through 152-4, pulleys 153-1 through 153-4, pulleys 154-1 through 154-4 and motors 15 The tactile pads 73-1 through 73-4 can be displaced and pressed against the skin 101 by various actuators, such as linear motors and electromagnetic solenoids, as well as motors 155-1 through 155-4.

The pads can be pressed against the skin 101.

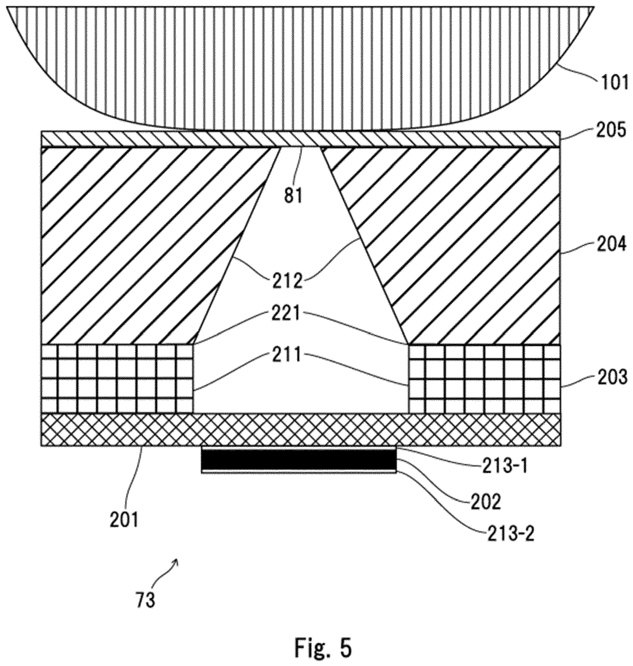

Next, an example of the details of the configuration of the tactile sensation generating pad 73 will be described. Figure 5 is an enlarged cross-sectional view of the tactile sensation generating pad 73. The tactile sensation generating pad 73 is composed of a vibrating plate 201, a vibration actuator 202, a fixed plate 203, a support member 204, and a vibration film 205. The diaphragm 201 is an example of a vibrating member and is vibrated by the vibration actuator 202. The diaphragm 201 is formed in a plate or thin film shape from an elastic material such as silicon.

The vibration actuator 202 is an example of a vibratory means and contacts the diaphragm 201 to vibrate the diaphragm 201. The vibration actuator 202 consists of piezoelectric elements such as PZT (lead zirconate titanate), PLZT (La-modified lead zirconate titanate), PMN-PT (lead magnesium niobate/titanate), AlN (aluminum nitride) or ScAlN (scandium aluminum nitride). (scandium aluminum nitride) and other piezoelectric elements. For example, the vibration actuator 202 is formed as a flat plate such as circular, oval, triangular, rectangular, or square. For example, the vibration actuator 202 is formed in a rectangular shape. The vibration actuator 202 stretches or bends when voltage is applied to the electrodes 213-1 and 213-2, which are provided on opposite sides, respectively. The vibration actuator 202 vibrates when an alternating voltage is applied to the electrodes 213-1 and 213-2. The vibration actuator 202 resonates and vibrates at a predetermined frequency when a voltage of a predetermined frequency is applied to the electrodes 213-1 and 213-2.

The fixing plate 203 is formed from an elastic material such as silicon. The fixing plate 203 is formed in the form of a plate with a greater thickness compared to the thickness of the diaphragm 201. The fixing plate 203 is provided between the support member 204 and the diaphragm 201 and is formed of a material having a smaller hardness compared to the hardness of the support member 204.

The support member 204 is formed of a material having a predetermined strength and a predetermined hardness, such as a plastic or other resin, a composite material such as a carbon composite, or a lightweight metal such as an aluminum alloy, titanium alloy or magnesium alloy. The support member 204 is formed in the shape of a plate. The thickness of the support member 204 is thicker than the thickness of the diaphragm 201 or the vibration film 205. The vibrating film 205 is a thin film formed of an elastic material such as silicon. The vibrating film 205 is formed thinner compared to the thickness of the diaphragm 201.

The support member 204 supports the vibration film 205 on the side surface in contact with the skin 101, and supports the fixed plate 203, the vibration plate 201, and the vibration actuator 202 on the side surface opposite the side surface in contact with the skin 101. The support member 204 supports the diaphragm 201 via the fixed plate 203 on the surface opposite the surface in contact with the skin 101. In other words, in the tactile sensation generating pad 73, the vibrating film 205, the support member 204, the fixed plate 203, the vibrating plate 201, and the vibration actuator 202 are arranged to be stacked in this order when viewed from the side in contact with the skin 101.

The support member 204 has holes 212 that penetrate from the side that supports the vibration film 205 to the side that supports the fixed plate 203, the vibration plate 201, and the vibration actuator 202. The hole 212 is open as an aperture 81 on the side of the support member 204 that supports the vibration film 205. The hole 212 is open as aperture 221 on the side of the face of the support member 204 that supports the fixed plate 203, the vibrating plate 201 and the vibration actuator 202. The area of aperture 81 is narrower than the area of aperture 221. For example, the hole 212 can be conical in shape with the face of the aperture 221 as a conical surface. For example, the hole 212 can be pyramidal, such as a square pyramid, such as a four-sided pyramid or a six-sided pyramid.

The fixing plate 203 has a hole 211 of the same cross-sectional shape as the aperture 221. The position of the opening of hole 211 in fixing plate 203 is aligned with the position of aperture 221. The aperture 221 and the hole 211 are provided so that the edge of the aperture 221 is adjacent to the edge of the hole 211. In other words, the fixed plate 203 has a hole 211 of the same cross-sectional shape as the aperture 221, which opens at the position of the aperture 221. In this way, the hole 212 and the hole 211 can be connected so that all of the apertures 221 overlap all of the openings of the apertures 221.

The vibrating film 205 is supported by the support member 204 so as to close the opening 81. For example, the portion of the support member 204 outside the edge of the aperture 81 is attached to the vibration film 205 over the entire edge of the aperture 81. The vibrating plate 201 is fixed to the fixing plate 203 so as to close the opening of the hole 211 in the fixing plate 203. For example, on the face of the fixing plate 203, on the side of the diaphragm 201, the outside of the edge of the opening of the hole 211 is attached to the diaphragm 2015 over the entire circumference of the edge of the opening of the hole 211. The fixing plate 203 can also be said to be provided between the support member 204 and the diaphragm 201, and the diaphragm 201 is supported by the support member 204 so that the diaphragm 201 closes the aperture 221 through the hole 211 in the fixing plate 203.

The vibration actuator 202 is installed in the position where the diaphragm 201 is closing the aperture 221. In this way, the diaphragm 201 can vibrate to a greater extent.

When the vibration actuator 202 vibrates the vibrating plate 201, the portion of the vibrating plate 201 at the position where the aperture 221 is closed vibrates. Since the area of aperture 81 is narrower than the area of aperture 221, the vibration of the portion of vibrating plate 201 at aperture 221 will be transmitted to the portion of vibrating film 205 that closes aperture 81 so that the amplitude of the vibration will be larger. In other words, the vibration of the portion of the diaphragm 201 at the aperture 221 is transmitted to the portion of the vibrating film 205 closing the aperture 81 so that the pressure of the vibration is increased. The vibration of the portion of the vibrating plate 201 at the aperture 221 is transmitted to the portion of the vibrating film 205 closing the aperture 81 so that the amplitude of the vibration is increased by the inverse of the ratio of the area of the aperture 81 to the area of the aperture 221.

In other words, the vibration of the diaphragm 201 at aperture 221 is concentrated on the vibrating film 205 at aperture 81 by means of hole 212. It can be said that the vibration of the diaphragm 201 at the aperture 221 is amplified by the hole 212.

As described above, the vibrating film 205 at aperture 81 can be vibrated more strongly. In other words, the vibrating film 205 at aperture 81 can be vibrated with a larger amplitude. In other words, the vibrating film 205 can be vibrated more strongly in a smaller area. By making aperture 81 a smaller area, it is possible to pseudo-transmit stronger vibration to a single point.

Skin 101 has a number of receptors for sensing vibration, shape, and shear forces. When the tactile generating pad 73 contacts the skin 101 and transmits vibrations, the receptors in the skin 101 are activated to generate tactile and pressure sensations. In other words, the tactile generating pad 73 uses sound pressure waves and pressure to generate tactile and pressure sensations by activating receptors in human skin 101 and the deep sensory receptors joint receptors and slow skin sensory receptors (Ruffini endings) using vibration and pressure stimulation.

As described in Figures 2 through 4, with the tactile sensation generating pad 73 pressed against the skin 101 by the air actuator 72, cam 122 and motor 123, or wire 152, pulley 153, pulley 154 and motor 155, the vibration actuator 202 vibrates the vibration plate 201 to to vibrate the vibrating film 205 at the aperture 81, the receptors on the skin 101 can be activated to generate tactile and pressure sensations. For example, when the tactile sensation generating pad 73 is pressed against the skin 101 with relatively weak force and the vibrating film 205 at the aperture 81 is vibrated, the Merkel disc, Meissner bodies and Pachini bodies near the surface of the skin 101 among the receptors of the skin 101 are activated to generate tactile sensations. For example, when the tactile sensation-generating pad 73 is pressed against the skin 101 with greater force, vibrating the vibrating film 205 at aperture 81 activates the Ruffini endings and the deep sensory receptors arthrodesis in the dermis of the skin 101 among the receptors of the skin 101 to generate a pressure sensation.

In the tactile sensation generating pad 73, a plurality of units comprising the vibrating plate 201 at aperture 221, the vibration actuator 202, the hole 211 in the fixed plate 203, the hole 212 in the support member 204, and the vibration film 205 at aperture 81 are formed. In the tactile sensation generating pad 73, the distance between each of the units comprising the vibrating plate 201 at the aperture 221, the vibration actuator 202, the hole 211 of the fixing plate 203, the hole 212 of the support member 204 and the vibration film 205 at the aperture 81 is less than the two-point discrimination distance of the human skin 101 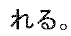

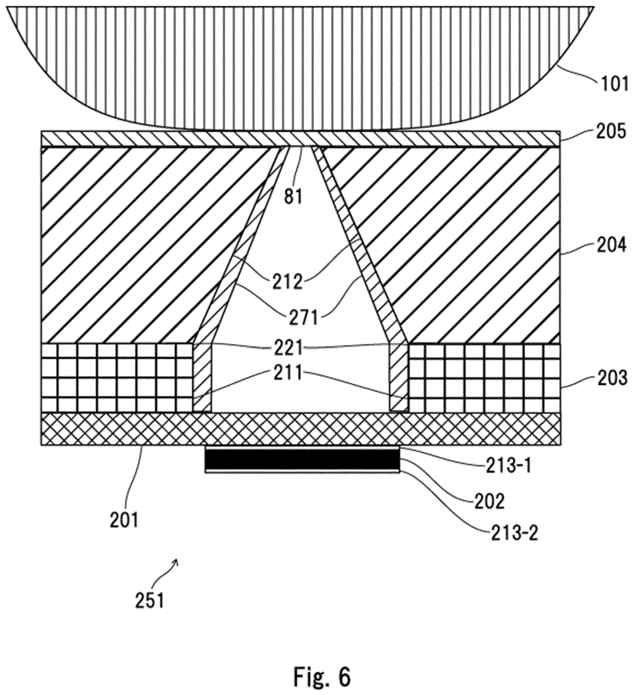

Next, an example of the details of the configuration of the tactile sensation generating pad 251, which constitutes the tactile sensation generating pad group 21, will be described. Tactile sensation generating pad 251 is used in tactile sensation generating pad group 21 in the same way as tactile sensation generating pad 73. Figure 6 is an enlarged cross-sectional view of the tactile sensation generating pad 251, showing an enlarged part of the cross section of the pad 251. In Fig. 6, parts similar to those shown in Fig. 5 are marked with the same symbols, and their descriptions are omitted.

The tactile sensation generating pad 251 is composed of a vibrating plate 201, a vibration actuator 202, a fixing plate 203, a support member 204, a vibration film 205, and a film 271. The film 271 is formed as a thin film on the inner surface of the hole 212 of the support member 204 and the hole 211 of the fixed plate 203. The coating 271 is formed of a hard or dense material such as tungsten, nickel, ceramic or aluminum. The coating 271 is molded of a material with a greater density than the density of the support member 204. More particularly, the coating 271 is molded of a material of greater hardness compared to the hardness of the support member 204 or of greater density compared to the density of the support member 204. For example, the coating 271 is molded of a material with a smaller grain size (particle size).

In this way, the vibration of the vibrating plate 201 at the aperture 221 is reflected by the inner surface of the hole 212 in the support member 204, while gathering at the portion of the vibrating film 205 at the aperture 81, allowing more efficient transmission of vibration. In other words, by providing the coating 271, the vibration of the diaphragm 201 at the aperture 221 can be transmitted to the portion of the vibration film 205 at the aperture 81 with less loss by reducing the vibration that is transmitted to the support member 204 or the fixed plate 203 and then attenuated.

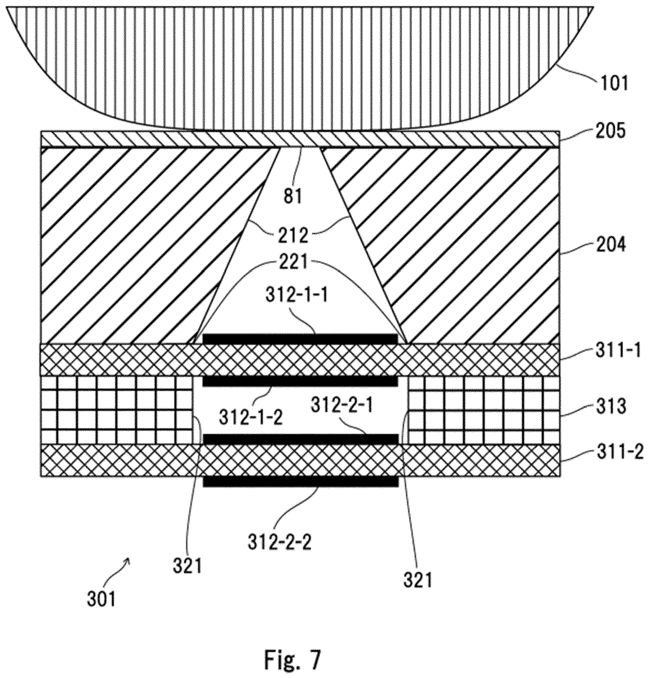

Next, an example of the details of the configuration of the tactile sensation generating pad 301 that constitutes the tactile sensation generating pad group 21 will be described. Tactile sensation generating pad 301 is used in tactile sensation generating pad group 21 in the same way as tactile sensation generating pad 73. Figure 7 is an enlarged cross-sectional view showing an enlarged part of the cross-section of the tactile sensation generating pad 301. In Fig. 7, parts similar to those shown in Fig. 5 are marked with the same symbols, and their descriptions are omitted.

The tactile sensation generating pad 301 comprises a support member 204, a vibration film 205, piezoelectric polymer films 311-1 and 311-2, and a spacer 313. In the tactile sensation generating pad 301, the vibrating film 205, the support member 204, the piezoelectric polymer film 311-1, the spacer 313, and the piezoelectric polymer film 311-2 are arranged to be stacked in this order when viewed from the side in contact with the skin 101. The piezoelectric polymer film 311-2 is arranged so that it is stacked in this order.

Piezoelectric polymer films 311-1 and 311-2 are piezoelectric piezoelectric materials such as PVDF (polyvinylidene fluoride), VDF-TrFE (polyvinylidene fluoride-trifluorotylene copolymer) or poly-L-lactic acid, respectively. Polymer actuators are piezoelectric polymer actuators. Piezoelectric polymer films 311-1 and 311-2 are stretched and polarized, respectively. Piezoelectric polymer films 311-1 and 311-2 can each be driven by a relatively low voltage. The piezoelectric polymer films 311-1 and 311-2 each vibrate on their own when a predetermined voltage is applied.

The spacer 313 is formed from an elastic material such as silicon. The thickness of the spacer 313 is at least twice the amplitude of the piezoelectric polymer films 311-1 and 311-2. The spacer 313 has a hole 321 of the same cross-sectional shape as the aperture 221 of the hole 212 in the support member 204. The position of the opening of the hole 321 in the spacer 313 is aligned with the position of the opening 221 across the piezoelectric polymer film 311-1. In other words, the aperture 221 and the hole 321 are provided so that the edge of the aperture 221 and the edge of the hole 321 are adjacent to each other across the piezoelectric polymer film 311-1.

The piezoelectric polymer film 311-1 is sandwiched between the support member 204 and the spacer 313 and arranged to close the aperture 221 of the hole 212 in the support member 204 and the hole 321 in the spacer 313. The piezoelectric polymer film 311-2 is disposed on the side of the spacer 313 opposite the side of the spacer 313 that is in contact with the support member 204 so as to close the hole 321 of the spacer 313.

Electrodes 312-1-1 and 312-1-2 are each provided on opposite sides of piezoelectric polymer film 311-1, facing each other. The electrodes 312-1-1 and 312-1-2 are arranged to fit between the aperture 221 of the hole 212 in the support member 204 and the hole 321 in the spacer 313, respectively.

When voltage is applied to electrodes 312-1-1 and 312-1-2, the portion of piezoelectric polymer film 311-1 The portion of piezoelectric polymer film 311-1 that is sandwiched between electrodes 312-1-1 and 312-1-2, i.e., the portion that fits between aperture 221 of hole 212 of support member 204 and aperture 321 of spacer 313 and closes the aperture 221 and aperture 321 The portion that closes the apertures of the support member 312-1-1 and the spacer 313 is stretched or flexed. When an alternating voltage is applied to electrodes 312-1-1 and 312-1-2, the portion of piezoelectric polymer film 311-1 that closes the apertures 221 and holes 321 The portion of the piezoelectric polymer film 311-1-1 that closes the openings vibrates. When a voltage of a predetermined frequency is applied to electrodes 312-1-1 and 312-1-2, the portion of the piezoelectric polymer film 311-1 that closes the apertures 221 and the portion of the piezoelectric polymer film 311-1 that closes the openings of apertures 221 and 321 resonate and vibrate at a predetermined frequency.

Electrodes 312-2-1 and 312-2-2 are each provided on opposite sides of piezoelectric polymer film 311-2, facing each other. Electrode 312-2-1 is positioned to fit into hole 321 of spacer 313.

When voltage is applied to electrodes 312-2-1 and 312-2-2, the portion of piezoelectric polymer film 311-2 that -2-1 and electrode 312-2-2, i.e., the portion that fits into hole 321 of spacer 313 and closes the opening of hole 321, expands and contracts or bends. When an alternating voltage is applied to electrodes 312-2-1 and 312-2-2, the portion of piezoelectric polymer film 311-2 that closes the opening of hole 321 The portions vibrate. When a voltage of a predetermined frequency is applied to electrodes 312-2-1 and 312-2-2, the portion of the piezoelectric polymer film 311-2 that closes the opening of hole 321 vibrates. apertures of the piezoelectric polymer film 311-2 resonate and vibrate at a predetermined frequency.

By vibrating the piezoelectric polymer films 311-1 and 311-2 in-phase, stronger vibrations can be transmitted to the vibrating film 205.

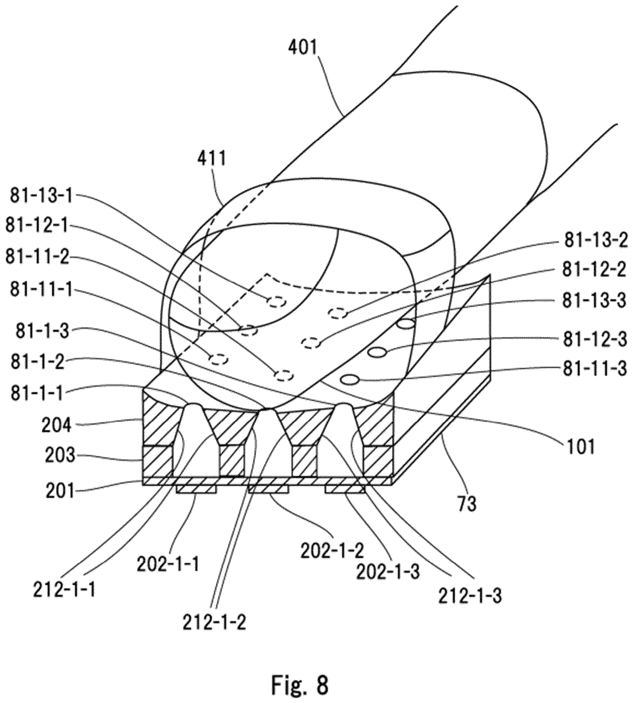

Next, an example of the use of the tactile sensation generating pad 73 as a device worn on the finger abdomen will be described. Figure 8 shows the state of the tactile sensation generating pad 73 attached to the finger abdomen. The tactile sensation generating pad 73 is attached to the finger 401 with a fixing band 411 so that the side of the vibrating film 205 contacts the skin 101 of the finger abdomen of the finger 401.

For example, the tactile sensation generating pad 73 as a device to be worn on the finger abdomen is approximately the same size as the finger 401 and its thickness is between 3 mm and 5 mm.

Vibration actuators 202-1-1 through 202-1-3, vibration actuators 202-11-1 through 202, vibration actuators 202-1-1 through 202-1-3 (not shown), vibration actuators 202-12-1 through 202-12-3 (not shown), and vibration actuators 2 when any of 02-13-1 through 202-13-3 (not shown) vibrates the vibrating plate 201, apertures 81-1 81-1-1 through 81-1-3, 81-11-1 through 81-11-3, 81 81-12-1 through 81-12-3 and 81-13-1 through 81-13-3. 3, any of which vibrate to generate stimulation and transmit the stimulation to the desired part of the skin 101 of the finger abdomen.

Thus, the tactile sensation-generating pad 73 can be used alone as a skin stimulation device.

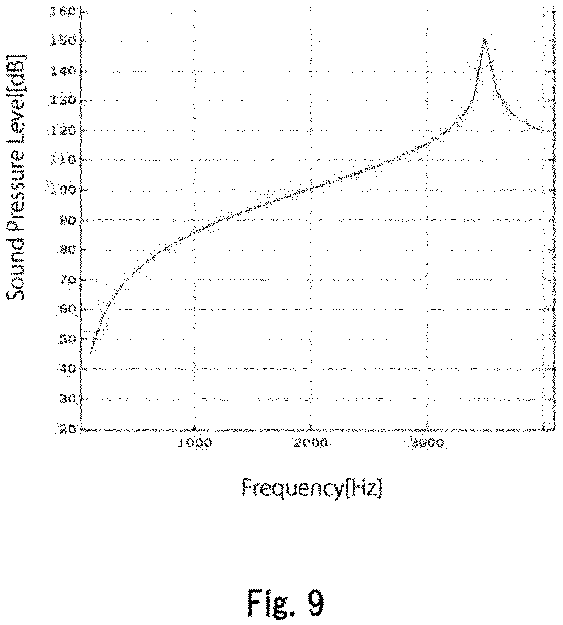

Figure 9 shows an example of the resonance characteristics of the vibration actuator 202 or piezoelectric polymer films 311-1 and 311-2. As shown in Figure 9, the vibration actuator 202 or piezoelectric polymer films 311-1 and 311-2 resonate at about 3500 Hz. The sound pressure from the vibration actuator 202 or piezoelectric polymer films 311-1 and 311-2 is very high at about 3500 Hz.

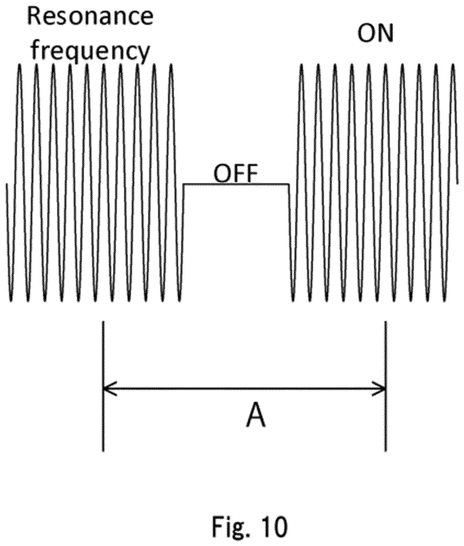

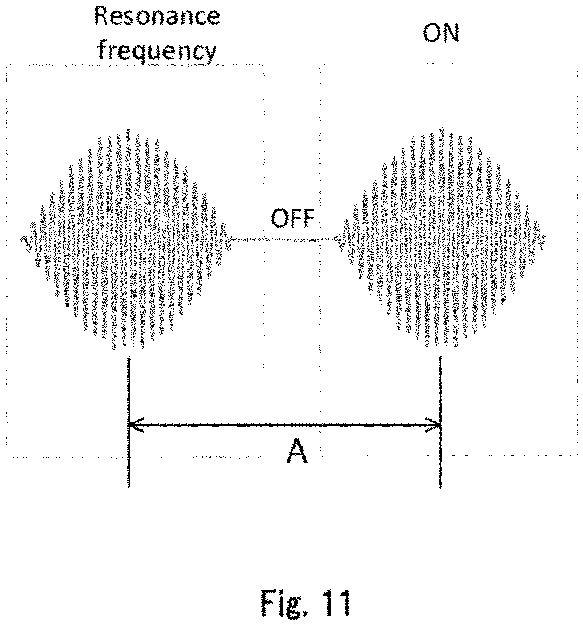

Next, the driving of the tactile generation pad 73, tactile generation pad 251 or tactile generation pad 301 by the voltage supplied from the amplifier 35 is explained. Figures 10 and 11 show examples of waveforms of voltages supplied from the amplifier 35 to the tactile sensation generating pad 73, tactile sensation generating pad 251 or tactile sensation generating pad 301. As shown in Figs. 10 and 11, burst signal-like voltages are supplied from amplifier 35 to tactile generation pad 73, tactile generation pad 251 or tactile generation pad 301. In other words, the amplifier 35 turns on and off at predetermined cycles, and during the off period, the voltage is approximately 0 V. During the on period, a sine wave of predetermined frequency with predetermined voltage amplitude is supplied to the tactile generator pad 73, the tactile generator pad 251, or the tactile generator pad 301. For example, from amplifier 35, a sine wave of the frequency at which vibration actuator 202 or piezoelectric polymer films 311-1 and 311-2 resonate is supplied to tactile generation pad 73, tactile generation pad 251 or tactile generation pad 301. For example, the amplifier 35 supplies a sine wave of 3500 Hz to the tactile generation pad 73, the tactile generation pad 251 or the tactile generation pad 301 during the period when it is on.

The frequency at which the burst signal-like voltage supplied from amplifier 35 is turned on or off is between 30 Hz and 300 Hz. In other words, the on/off period of the burst signal voltage supplied from amplifier 35 is from 3 msec to 30 msec, as shown in Figure 10 and Figure 11, A.

The perceived frequency of stimulation of human skin 101 is relatively low, ranging from tens of Hz to 300 Hz. During the period when the burst signal-like voltage supplied from amplifier 35 is on, a sine wave of the frequency at which vibration actuator 202 or piezoelectric polymer films 311-1 and 311-2 resonate is supplied to tactile generating pad 73, tactile generating pad 25 1 or tactile generating pad 301, so stronger stimulation can be applied to the skin 101. In addition, since the burst signal-like voltage is turned on or off in the range of the perceived frequency of stimulation of human skin 101, the vibration is turned on or off in the range of the perceived frequency of stimulation of human skin 101, making it easier to perceive the presence or absence of stimulation. This allows for a low-frequency tactile sensation on human skin 101.

As shown in Figure 10, for example, the envelope of the burst signal-like voltage supplied from amplifier 35 to tactile generator pad 73, tactile generator pad 251 or tactile generator pad 301 is made square wave-like with line symmetry in the positive and negative directions of amplitude with respect to a level of 0 V in amplitude.

As shown in Figure 11, for example, the envelope of the burst signal-like voltage supplied from amplifier 35 to tactile generation pad 73, tactile generation pad 251 or tactile generation pad 301 is line-symmetrical sinusoidal in the positive and negative directions of amplitude with respect to the 0 V level of amplitude.

The voltage supplied from the amplifier 35 to the vibrating plate 201 or piezoelectric polymer films 311-1 and 311-2 of the tactile generating pad 73, pad 251 or pad 301 is not limited to a burst signal form. The voltage supplied to the diaphragm 201 or piezoelectric polymer films 311-1 and 311-2 is not limited to burst signals, but can also be a sinusoidal waveform that continues for a predetermined period of time or a constant voltage.

It was explained that a sine wave of a predetermined frequency at a predetermined voltage is supplied from the amplifier 35 to the tactile generator pad 73, tactile generator pad 251 or tactile generator pad 301 during the on period, but it may be a square wave, rectangular wave, triangular wave, saw blade wave or pulse, etc.

Thus, the diaphragm 201 or the piezoelectric polymer films 311-1 and 311-2 of the tactile generating pad 73, the tactile generating pad 251 or the tactile generating pad 301 are in the form of burst signals that are turned on or off at a period of 3 msec to 30 msec. When on, the diaphragm 201 or piezoelectric polymer films 311 - 1 and 311-2 are made to vibrate at a frequency that causes them to resonate.

Thus, a wearable device can apply a stimulus that causes tactile sensation to a desired part of the skin site at a desired time and for a desired period of time.

For example, stimuli can be generated to perceive object shapes and vibration patterns, such as edge and bump shapes.

Thus, the diaphragm 201 vibrates under excitation. The piezoelectric polymer film 311-1 vibrates by itself. The vibrating film 205 contacts the skin 101 and transmits vibration as stimulation. The support member 204 supports the vibrating film 205 on a first surface, which is a predetermined surface, and supports the vibrating plate 201 on a second surface opposite the first surface. The support member 204 has a hole 212 that penetrates from the first face to the second face, wherein the area of the opening 81 on the first face side is narrower than the area of the opening 221 on the second face side. The vibrating film 205 is supported by the support member 204 so as to close the aperture 81. The vibrating plate 201 is supported by the support member 204 so as to close the aperture 221 and is vibrated in the position where the aperture 221 is closed. The piezoelectric polymer film 311-1 is supported by the support member 204 so as to close the aperture 221 and vibrates by itself in the position in which the aperture 221 is closed.

In this way, the skin stimulation device can be used as a wearable device to apply stimulation causing tactile sensation to a desired part of the skin site at a desired time and for a desired duration.

A plurality of holes 212 can be formed in the support member 204.

The spacing of the plurality of holes 212 in the support member 204 can be less than the two-point identification threshold of human skin 101.

A vibration actuator 202 can be further provided to vibrate the diaphragm 201, which is in contact with the diaphragm 201 at the position where the diaphragm 201 is closing the aperture 221.

The vibration actuator 202 can be made to resonate at a given frequency.

The vibration actuator 202 can be a piezoelectric element.

A further piezoelectric polymer film 311-2, which vibrates by itself, can be provided between spacers 313 for the piezoelectric polymer film 311-1.

Piezoelectric polymer film 311-1 and piezoelectric polymer film 311-2 can be

The coating 271 can be formed on the inner surface of the hole 212 in the support member 204 with a material of greater density than the density of the support member 204.

A fixing plate 203 can be further provided between the support member 204 and the diaphragm 201, molded of a material of lesser hardness compared to the hardness of the support member 204, and having a hole 211 of the same cross-sectional shape as the aperture 221 opening at the location of the aperture 221.

When the vibrating film 205 contacts the skin 101 and transmits vibration as stimulation, an air actuator 72 can be further provided to displace the vibrating plate 201, vibrating film 205 and support member 204 to press them against the skin 101.

In this way, as a wearable device, it can apply stimulation causing tactile and pressure sensations to a desired part of the skin site at a desired time and for a desired duration.

The air actuator 72 can be pressed against the skin 101 by displacing the diaphragm 201, vibration film 205, and support member 204 by gas or liquid pressure.

The cam 122 as the pressing means can displace the diaphragm 201, vibrating film 205, and support member 204 to press them against the skin 101.

As a pressing means, the vibrating plate 201, vibrating film 205, and support member 204 can be displaced and pressed against the skin 101 by pulling with wire 152 via pulley 153.

A vibrating plate 201 that vibrates or vibrates by itself when vibrated, a vibrating film 205 that contacts the skin 101 and transmits vibration as a stimulus, a support member 204 that supports the vibrating film 205 on a first side, which is a predetermined surface, and supports the vibrating plate 201 on a second side opposite the first side and has a hole 2 2 that passes through the first side to the second side 12, including a support member 204 having a hole 212 formed wherein an area of the opening 81 on the first side is narrower than an area of the opening 221 on the second side, the vibration film 205 being supported by the support member 204 so as to close the opening 81, the diaphragm 201 being supported by the support member 204 so as to close the opening 221 The vibrating plate 201 of the tactile generating pad 73, which is a skin stimulation device that is vibrated or vibrates by itself in the position of closing the aperture 221, can be driven by vibrating the vibrating plate 201 at a frequency that resonates the vibrating plate 201 when on, in the form of a burst signal that is turned on or off in a cycle of 3 msec to 30 msec. When on, the diaphragm 201 can be driven by vibrating it at a frequency that causes it to resonate.

In this way, the skin stimulation device, which is a wearable device, can apply stimulation causing tactile sensation to a desired part of the skin site at a desired time and for a desired duration.

The invention is not limited to the embodiments described above, and various changes can be made to the extent that they do not depart from the gist of the invention.

### Explanation of Signs

11 Tactile sensation presentation system, 21 Tactile sensation generating pad group, 31 Computer system, 32 Tactile sensation detection system, 33 Vibration frequency/pressure calculation unit, 34 Digital data/analog data conversion unit, 35 Amplifier, 41 Collision calculation unit, 42 Robot system, 52 Transducer, 53 Remote robot system 71 and 71-1 to 71-4 tactile sensation generating units, 72 and 72-1 to 72-4 air actuators, 73 and 73-1 to 73-4 tactile sensation generating pads, 81 81, 81-1-1 through 81-1-3, 81-2-1 through 81-2-3, 81-3-3, 81-4 81, 81-1-1 through 81-1-3, 81-3-1 through 81-3-3, 81-4-1 through 81-4-3, 81-11 81-4-1 through 81-4-3, 81-11-1 through 81-11-3, 81-12-1 through 81-12-3, and 81-13-1 through 81-13-1. 81-12-3 and 81-13-1 through 81-13-3 openings, 101 skin, 121 and 121-1 through 121-4 tactile sensation generating unit, 122 and 122-1 through 122-4 cams, 123 and 123-1 through 123-4 motors, 151 and 151 151 and 151-1 through 151-4 tactile sensation generating units, 152 and 152-1 through 152-4 wires, 153, 153-1 through 153-4, 1 54 and 154-1 through 154-4 pulleys, 155 and 155-1 through 155-4 motors, 201 diaphragm, 202, 202-1 through 202-1 202, 202-1-1 through 202-1-3, 202-11-1 through 202-11-3, 202 202, 202-1-1 through 202-1-3, 202-11-1 through 202-11-3, 202-12-1 through 202-12-3 and 202-13-1 through 202-13-3 -3 Vibration actuator, 203 Fixed plate, 204 Support member, 205 Vibration film, 211 and 212 Holes, 213-1 and 213-2 Electrodes, 221 Apertures, 271 Film, 311 311-1 and 311-2 piezoelectric polymer films, 313 spacers, 312-1-1, 312-1-2, 312 312-1-1, 312-1-2, and 312-2-2 electrodes, 401 finger, 411 fixation band

## Claims

1. A skin stimulation device that applies stimulation to the skin, comprising:
a first vibrating member that vibrates by being vibrated or vibrates by itself,
a vibration film that contacts the skin and transmits the vibration as stimulation, and
a first support member supporting the vibrating film on a first surface, which is a predetermined surface, and supporting the first vibrating member on a second surface opposite the first surface, wherein a first hole penetrating from the first surface to the second surface is formed, and, in the first hole, a first opening on the first surface has a narrower area than a second opening on the second surface.
wherein the vibration film is supported by the first support member so as to close the first opening, and
the first vibrating member is supported by the first support member so as to close the second opening and is vibrated or vibrates itself in the position of closing the second opening.

2. The skin stimulation device according to claim 1,
wherein a plurality of first holes are formed in first support member.

3. The skin stimulation device as recited in claim 2,
wherein the spacing between a plurality of first holes in first support member is less than the two-point identification threshold of human skin.

4. The skin stimulation device as according to claim 1,
further comprising vibratory means for vibrating the first vibrating member, which is provided in contact with the first vibrating member at the position where the first vibrating member is closing the second opening.

5. The skin stimulation device according to claim 4,
wherein the vibratory means resonates at a predetermined frequency.

6. The skin stimulation device according to claim 4,
wherein the vibratory means comprises a piezoelectric element.

7. The skin stimulation device according to claim 1,
further comprising a second vibrating member that vibrates by itself, which is provided between spacers with respect to the first vibrating member that vibrates by itself.

8. The skin stimulation device according to claim 7,
wherein the first vibrating member and the second vibrating member comprise a piezoelectric polymer film.

9. The skin stimulation device according to claim 1,
wherein a coating is formed on the inner surface of the first hole of the first support member with a material of greater density than the density of the first support member.

10. The skin stimulation device according to claim 1,
further comprising a second support member provided between the first support member and the first vibrating member, molded of a material of lesser hardness compared to the hardness of the first support member, and having a second hole of the same cross-sectional shape as the second opening at the location of the second opening.

11. The skin stimulation device according to claim 1,
further comprising pressing means for displacing and pressing first vibrating member, vibrating film and first support member against skin when vibrating film contacts skin and transmits vibration as stimulation.

12. The skin stimulation device according to claim 11,
wherein the pressing means displaces and presses the first vibrating member, the vibrating film and the first support member against the skin by gas or liquid pressure.

13. The skin stimulation device according to claim 11,
wherein the pressing means uses a cam to displace and press the first vibrating member, the vibrating film and the first support member against the skin.

14. The skin stimulation device according to claim 11,
wherein the pressing means displaces and presses the first vibrating member, the vibrating film and the first support member against the skin by pulling with a wire via a pulley.

15. A method for driving a skin stimulation device that applies stimulation to the skin, comprising:
vibrating a vibrating member of the skin stimulation device at a frequency in the form of a burst signal that is turned on or off in a cycle of 3 msec to 30 msec,
wherein the frequency resonate the vibrating member when the burst signal is turned on,
wherein the skin stimulation device comprises the vibrating member that vibrates or vibrates by itself when vibrated, a vibrating film that contacts the skin and
transmits vibration as stimulation, and a support member supporting the vibrating film on a first face, which is a predetermined face, and supporting the vibrating member on a second face opposite to the first face, a hole is formed in the support member, that the hole penetrates from the first face to the second face,
the area of the first opening of the hole on the first side is narrower than the area of the second opening of the hole on the second side, the vibration film being supported by the support member so as to close the first opening, the vibration member being supported by the support member so as to close the second opening, and
the vibrating member of the skin stimulation device is vibrated or vibrates by itself in a position in which the second opening is closed.
